# EUROPEAN PATENT APPLICATION

(11) **EP 2 394 631 A1**
(43) Date of publication of application: **14.12.2011**
(21) Application number: 10005891.6
(22) Date of filing: 08.06.2010
(51) Int. Cl.: A61K 8/19, A61K 8/25, A61K 8/31, A61K 8/55, A61K 8/92, A61Q 5/06, A61Q 5/12

(54) **Hair treatment composition**

(71) Applicant: KPSS-Kao Professional Salon Services GmbH, 64297 Darmstadt (DE)
(72) Inventor: Punsch, Britta, 01723 Kesselsdorf (DE); Cajan, Christine, 56130 Bad Ems (DE)
(74) Representative: Grit, Mustafa

(57) **Abstract**

Present invention relates to an aqueous composition comprising insoluble particles suspended therein for conditioning and matifying of hair. The first object of the present invention is an aqueous composition for hair comprising at least 20% by weight water, 10 to 60% by weight of at least one wax which is solid at 24°C, at least one emulsifying surfactant at a concentration in the range of 2 to 25% by weight and dispersed particles with a size below 200 µm measured with sieve analysis at a concentration of 1 to 35% by weight, wherein all values are calculated to total composition.

## Description

Present invention relates to an aqueous composition comprising insoluble particles suspended therein for conditioning, especially styling and matifying of hair.

Compositions for matifying hair have increasingly been used which are basically, emulsions and usually comprising a wax component suspended and or emulsified in an aqueous medium. Such compositions, however, do not provide optimal long lasting conditioning, especially styling and matifying effects and espacially, hair styling is laborious because of long drying time of the product on hair, especially when applied onto dry hair, without using hair drier. Furthermore, after drying hair, residues have always been visible on hair and hair has a gray shimmer which disturbs cosmetically appealing appearance of hair.

Therefore, there is a great need for a conditioning, especially styling and matifying composition with quick drying property on hair when applied onto dry hair without using hair drier and especially a composition which does not leave any visible residue in form of particles on hair after application and drying.

WO 2009/056398 A2 discloses a mat wax composition comprising wax components with a melting temperature above 40°C and specially treated solid particles with a particle size up to 1 µm. The document does not refer to quick drying properties and does not disclose any composition which may be taken as closely related to the present invention.

WO 01/74311 A2 is on leave-in hair conditioning composition comprising non-spherical micro-particles with a size less than 100 µm and a water swellable polymer. Document does not necessarily relate to a wax comprising composition and does not address matifying effect together with quick drying property and visibility of the particles after application and especially drying.

Present inventors have surprisingly found out that an aqueous composition comprising wax, surfactant and particles dispersed therein show excellent conditioning especially styling matifying and effects and composition dries quickly and especially no visible particles have been observed on the hair after application and drying. It has furthermore been found out that the matifying and styling effects and also conditioning effect are long lasting.

Thus, the first object of the present invention is an aqueous composition for hair comprising at least 20% by weight water, 10 to 60% by weight of at least one wax which is solid at 20°C, at least one emulsifying surfactant at a concentration in the range of 2 to 25% by weight and dispersed particles with a size below 200 µm measured with sieve analysis at a concentration of 1 to 35% by weight, wherein all values are calculated to total composition.

Second object of the present invention is that the aqueous composition comprises inert non-swelling particles.

In a third object of the present invention, the aqueous composition comprises particles of at least one inert non-swelling inorganic compound.

Further object of the present invention is the use of the aqueous composition for conditioning, styling and matifying hair.

With the term matifying. it is meant that the hair shine is reduced so that intensity of the light reflected is considerably less, preferably no light reflection takes place.

The compositions of the present invention are used as leave in compositions, not rinsed off from hair after application. Thus, another object of the present invention is process for conditioning and/or styling and/or mattifying hair wherein the composition of the present invention is applied onto dry hair and optionally hair is dried.

The composition of the present invention is preferably provided to the users, consumers, in the form of a kit comprising more than one container, preferably 2 to 5, more preferably 2 to 4 and most preferably 2 to 3 containers wherein one of the containers comprises a composition of the present invention.

Aqueous composition of the present invention comprises at least 20% by weight water, calculated to total of the composition. Preferably, aqueous composition comprises water at a concentration of 20 to 80% by weight, more preferably 30 to 70% by weight and most preferably 35 to 60% by weight and in particular 35 to 55% by weight, calculated to total of the composition.

Composition of the present invention comprise at least one wax component at a concentration of 10 to 60% by weight, preferably 15 to 60% by weight, more preferably 15 to 50% by weight, most preferably 20 to 40% by weight calculated to total of the composition.

The term wax refers to any compound with a melting point above 20°C preferably above 30°C and more preferably 40°C. Suitable ones petrolatum, ozokerit, carnauba wax, paraffin, lanolin wax, candelila wax, bees wax, microcrystalline wax, cocoglyceride, fatty alcohols and their ethoxylated derivatives.

Further suitable ones are glycerin fatty acid esters with an acyl chain length of 14 to 24 C atoms which may be straight or branched and may have one or more substitutions, with a melting point above 40°C such as glyceryl stearate, palmitate, behenate etc. which may further be ethoxylted and/or propoxylated.

Further suitable wax components are fatty alcohols such as cetyl alcohol, stearyl alcohol, myristyl alcohol, behenyl alcohol and their mixtures.

Further suitable as wax components are ethoxylated fatty acids with an acyl chain length of 14 to 24 C atoms with a melting point above 40°C such as PEG-100 stearate.

Preferred wax components are beeswax, petrolatum, ozokerit, carnauba wax and their mixtures.

Composition of the present invention comprises at least one surfactant preferably selected from anionic and non-ionic surfactants.

Suitable sulphate surfactants are according to the general structure

R₁ (OCH₂CH₂)ₙ O SO₃ M

wherein R is an alkyl chain which may be saturated or unsaturated, straight or branched with 10 to 22 C atoms, preferably 12 to 20 and more preferably 12 to 18 and most preferably 12 to 16 C atoms, n is a number between 0 and 5 and M is a cation and preferably sodium, potassium or ammonium, more preferably sodium or ammonium. Suitable examples are alkyl sulphates such as sodium lauryl sulphate, sodium cetyl sulphate, sodium stearyl sulphate and alkyl ether sulphates such as sodium laureth sulphate, sodium myreth sulphate, sodium ceteth sulphate, sodium oleth sulphate, sodium steareth sulphate, ammonium laureth sulphate, ammonium myreth sulphate, ammonium oleth sulphate, ammonium ceteth sulphate, ammonium steareth sulphate.

Suitable surfactants of the carboxylate type are alkyl polyether carboxylic acids and the salts thereof of the formula

R₂ - (C₂H₄O)ₙ - O - CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Suitable anionic aminocarboxylate surfactants are also C₈-C₂₂-acyl aminocarboxylic acids or the water-soluble salts thereof, such as N-lauroyl glutamate, in particular as sodium salt, as well as, for example, N-lauroyl sarcosinate, N-C₁₂-C₁₈acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in mixture with the above-named anionic surfactants.

Additional anionic surfactants to mention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Particularly preferred anionic surfactants are phosphate surfactants and especially the ones according to general structure

(R₃(OC₂ H₄)ₓ(OC₃ H₆)yO)₃PO

wherein R₃ is an alkyl chain which may be saturated or unsaturated branched or straight with 8 to 22 C atoms, preferably 10 to 18 C atoms and more preferably 12 to 16 C atoms, x and y are in the range of 0 to 10 with the condition x+y is more than 0. Suitable and most preferred anionic phosphate surfactant is Trilaureth-4 phosphate and its salts.

Suitable non-ionic surfactants are alkyl polyglucosides of the general formula

R₁₂ - O -(R₁₃O)ₙ O-Zₓ

wherein R₁₂ is an alkyl group with 8 to 18 carbon atoms, R₁₃ is an ethylene or propylene group, Z is a saccharide group with 5 to 6 carbon atoms, n is a number from 0 to 10 and x is a number between 1 and 5. Examples are.decyl polyglucoside, cocoyl polyglucoside both are commercially available.

Further nonionic surfactant components are, for example, long-chain fatty acid mono-and dialkanolamides, such as coco fatty acid monoethanolamide and myristic fatty acid monoethanolamide.

Further additionally useful non-ionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 50, preferably about 10 and about 30.

Among the non-ionic surfactants mentioned above fatty alcohol ethoxylates are the most preferred ones. Above mentioned non-ionic surfactants can also be used as mixture of one category such as several ethoxylated fatty alcohols or several categories such as mixture of alkyl polyglucoside and ethoxylated fatty alcohol.

Total surfactant concentration varies between 2 and 25%, preferably 3 and 25%, and more preferably 5 and 20% and most preferably 7.5 and 15% by weight, calculated to total composition.

Compositions suitably comprise at least one anionic and at least one non-ironic surfactants. The more preferred are anionic surfactants and especially those anionic surfactants with sulphate and phosphate end groups. In particularly preferred from of the present invention, compositions comprise at least one phosphate surfactant of the above structure, and in particular trilaureth-4 phosphate.

Composition of the present invention comprises dispersed particles with a size below 200 µm measured with sieve analysis at a total concentration of 1 to 35% by weight, preferably 2 to 30%, more preferably 3 to 25% and most preferably 5 to 20% by weight, calculated to total composition.

In a more preferred from of the present invention, composition comprises a first dispersed particles component with a size below 200 µm measured with sieve analysis and a second dispersed particles with the same size specification which is selected from the ones inert, non-swelling inorganic powder compounds. Suitable ones are calcium salts and especially preferred is Calcium sulphate.

Suitable examples to the first dispersed particles are silicium dioxide, starch such as wheat, rice, potato and corn, and titan dioxide and their mixtures.

In a further preferred from of the present invention composition comprises the second powder component at a concentration higher than the first dispersed particles. Preferably the weight ratio of first to second powder component is at least 1:1.01 and more preferably at least 1:1.1 and most preferably at least 1:1.5 and in particular at least 1:2.

Compositions of the present invention may comprise additional compounds found customarily in the cosmetic compositions.

Compositions may comprise at least one hair styling polymer selected from anionic, cationic, non-ionic and amphoteric ones at a concentration of 0.1 to 25%, preferably 1.5 to 20%, more preferably 2.5 to 15 and most preferably 4 to 15% by weight calculated to total composition.

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luvisko) VA 55, VA 64; Plus from BASF AG.

Natural non-ionic polymers are as well suitable for the composition of the present invention. Those are such as cellulose, chitosan, guar gum and their derivatives.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g., the butyl methacrylate copolymer "Yukaformer® Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (metly-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers Known from US-A 3,927,199.

Suitable anionic polymers alone or in combination with non-ionic polymers are vinyl - alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert,-butyl acrylamide, copolymers of the type "Ultrahold®"; vinyl pyrrolidone/vinyl acetate/itaconic acid copolymers, acrylic acid/acrylamide copolymers or the sodium salts thereof of the type "Reten®"; etc.

Further suitable anionic polymers are Acrylate copolymers available under trade name Salcare SC 81, PEG/PPG 25/25 dimethicone / acrylate copolymer available under trade name Luvigel Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66

Composition of the present invention can comprise cationic polymers alone or in combination with non-ionic polymer. Those are cationic cellulose type polymers know as Polymer JR type from Amerchol such as Polyquaternium-10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, those cationic polymers known with their CTFA category name Polyquaternium may as well be added into the compositions of the present invention. Typical examples of those are Polyquaternium-6, Polyquaternium-7, Polyquaternium-10, Polyquaternium-11, Polyquaternium-16, Polyquaternium-22 and Polyquaternium-28, Polyquatemium-30, Polypaternium-37, Polyquaternium-36, Polyquatemium-46, Polyquaternium-24, Polyquaternium-67, and Polyquaternium-72.

In this context, reference is also made to the cationic polymers disclosed in DE 25 21 960, 28 11 010, 30 44 738 and 32 17 059, as well as to the products descried in EP-A 337 354 on pages 3 to 7, It is also possible to use mixtures of various cationic polymers.

The cationic polymers also include the quaternized products of graft polymers from organopolysiloxanes and polyethyl oxazolines described in EP-A 524 612 and EP-A 640 643. Among these especially preferred is the compound know with the INCI name Polysilicone-9.

In a further embodiment of the present invention, the composition can comprise at least one synthetic or natural oil. In principal, any oil allowed for cosmetic use is suitable for the compositions of the present invention.

Oils are those of synthetic and of natural ones. Natural oils are in principal any triglyceride suitable for cosmetic use. Non-limiting examples are avocado oil, coconut oil, palm oil, sesame oil, peanut oil, sunflower oil, almond oil, peach kernel oil, wheat germ oil, macadamia nut oil, night primrose oil, jojoba oil, castor oil, of also olive oil, soya oil, and the derivatives thereof. Mineral oils such as paraffin oil and Petrolatum are suitably contained within the scope of the present invention, it should as well be noted that compositions of the present invention can contain mixture of one or more natural oils and mineral oil.

Further, suitable synthetic oil components are in particular fatty alcohol fatty acid esters such as isopropyl myristate, palmitate, stearate and isostearate, oleyl oleate; isocetyl stearate, hexyl laurate, dibutyl adipate, dioctyl adipate, myristyl myristate, oleyl erucate, polyethylene glycol and polyglyceryl fatty acid esters, cetyl palmitate, etc.

Synthetic ones are those of silicone oils. Here again any silicone oil either volatile and/or non-volatile is suitable for the compositions of the present invention. Preferred silicone oils are non-volatile silicone oils known with their INCI name as dimethicone and dimethiconol. Volatile silicone oils such as cyclomethicones may be used in combination with non-volatile silicones and/or other wax and/or oils mentioned above. Commercially, they are available from various companies for example Dow Corning with the known DC series, Wacker Chemie and Toray silicones. All commercially available non volatile silicones are suitable in the compositions of the present invention. Examples to those are DC 200 series, DC1401, DC 1403, DC 1501 and DC 1503. Furthermore, aminated silicones such as amodimethicone and arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane can be advantageously comprised in the compositions of the present invention.

Concentration of one or more oil in the compositions of the present invention may be between 1 and 25%, preferably 1.5 and 20% and more preferably 2 and 15% by weight calculated to total composition.

The composition of the present invention can comprise polyols at a concentration of 0.5 to 15%, preferably 1 to 10%, more preferably 2 to 5% by weight calculated to the total composition. The most preferred ones are glycerine, propylene glycols, butylene glycol and hexylene glycol.

The compositions according to the invention may also comprise further agents, such as proteins, for example bamboo protein, and protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin^{®}".

Additional natural plant extracts can as well form part of the compositions of the present invention. Those are incorporated usually in an amount of about 0.01 % to about 5 %, preferably 0.05 % to 3.5 %, in particular 0.1 % to 2 % by weight, calculated as dry residue thereof to the total compositions Suitable aqueous (e.g. steam-distilled) alcoholic or hydro-alcoholic plant extracts known per so are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot., currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "'Extrappn®" products, "Herbasol^{R} ", "Sedaplant^{R"} and "Hexaplant^{R}". Extracts and the preparation thereof are also described in "Hagers Handbuch der pharmazeutischen Praxis". 4^{th} Ed.

Compositions of the present invention may contain UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnarnic acid and the esters and salts thereof, 4-methoxycinnamic acid and the esters and salts thereof, salicylic acid and the esters and salts thereof. 2.4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzophenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, and/or polysiliocne-15.

The suitable amount of the UV-absorber ranges from about 0.01 % to 1 % by weight. calculated to the total composition. Attention should be paid to the stability and solubility especially when using UV filter as salts, e.g. anionic UV filter salts.

The compositions of the present invention can contain one or more organic solvents within the scope of the invention, Suitable ones are ethanol, propanol, isopropanol, isopentane, n-pentane, n.hexane, dimethoxymethane, benzyl alcohol, benzyloxyethanol, alkylene carbonates such as ethylene carbonate and propylene carbonate, phenoxyethanol, butanol, isobutanol, cyclohexane, cyclohexanol, ethylenecarbonate, ethyleneglycol monoethylether, ethylene glycol monobutyl ether, ethylene glycol monophenyl ether, 1-phenylethylalcohol, 2-phanylethylelcohol, o-methoxyphenol. The most preferred organic solvents are ethanol, isopropanol and propanol. Concentration of solvents is in the range of 0 to 20% and preferably 1 to 15%, more preferably 1 to 10% and most preferably 2 to 10% by weight calculated to total composition.

In a further embodiment of the present invention, the compositions comprise at least one direct dye for colouring hair. Suitable direct dyes are cationic, anionic. neutral dyes and their mixtures as available commercially from various suppliers and used mainly in semipermanent hair coloration.

Non-limiting examples to cationic dyes are Basic Blue 6, Basic Blue 7. Basic Blue 9, Basic Blue 26, Basic Blue 41, Basic Blue 99, Basic Brown 4, Basic Brown 16, Basic Brown 17, Basic Orange 31, Natural Brown 7, basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 37.

Further suitable direct dyes are anionic dye. Suitable non-limiting examples are Acid Black 1, Acid Blue 1, Acid Blue 3, Food Blue 5, Acid Blue 7, Acid Blue 9, Acid BLue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155. Arid, Red 180, Acid Violet 9, Acid Violet 43, Acid Violet 49, Acid Yellow 1, Acid Yellow 23, Acid Yellow 3, Food Yellow No.8. D&C Brown No.1, D&C Green No. 5, D&C Green No.8, D&C Orange Na.4, D&C Orange No. 10, D&C Orange No.11, D&C Red No.21. D&C Red No. 27, D&C Red No. 33, D&C Violet 2, D&C Yellow No. 7,D&C Yellow No. 8, D&C Yellow No. 10, FD&C Red 2, FD&C Red 40, FD&C Red No.4. FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No,2. HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1. HC Brown No.2, HC Green No.1, HC Orange No.1, HG. Orange No.2, HC Orange No,3, HC Orange No.5, HC Red BN, HC Red No. 1, HC Red No.3, HC Red No.7, HC Red No.8, HC Red No.9, HC Red Na.10, HC Red No.11. HC Red No.13. HC Red No.54, HC Red NO.14, HC Violet BS, HC Violet No.1, HC Violet No.2, HC Yellow No.2, HC Yellow No.4, HC Yellow No.5. HC Yellow No.6, HC Yellow No.7, HC Yellow. No.8. HC Yellow No.9, HC Yellow Na.10, HC Yellow No.11, HC Yellow No,12, HC Yellow No.13, HC Yellow No.14, HC Yellow No.15, 2- Aminn-6-chloro-4-nitrophenal, picramic acid, 1,2-Diamino-4-nitrobenzol. 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention for example henna (red or black), alkanna root, laccaic acid, indigo, logwood powder, madder root and rhubarb powder, etc.

It should be noted that the above dyestuffs are also suitable for use in mixture. When using direct dyes of various categories, their compatibility must be checked.

The above mentioned dyestuffs are also used especially the anionic ones for product colouring at reduced concentrations.

Concentration of direct dyes in the compositions of the present invention is within the range of 0.001 to 5%, preferably 0.01 to 3% and more preferably 0.05 to 2%. and most preferably 0.1 to 1% by weight calculated to total composition, calculated to total composition.

In case an aerosol type of styling product is designed then composition of the present invention comprises additionally at least one propellant.

Compositions of the present invention may comprise at least one propellant at a concentration of 5 to 60%, preferably 5 to 50% more preferably 10 to 50% by weight calculated to total composition. Suitable propellants are lower alkanes such as n-butane, i-butane, propane, butane or their mixtures, as well as dimethylether (DMF) either alone or in mixture with lower alkanes. Further suitable propellants are fluorinated hydrocarbons such as 1,1-difluoro ethane or tetrafluoroethane or their mixtures with each other, carbon dioxide and nitogen or their mixtures with the above mentioned propellants. Preferred are lower alkanes such as propane, butane, n-butane, i-butane, and their mixtures and their mixture with DME at a alkane to DME weight ratio 10:1 1 to 1:10.

Furthermore compositions of the present invention can comprise all substances customarily found In such preparations. Examples of such substances are complexing agents, preservatives, fragrances, etc.

The following examples are to illustrate the invention but not to limit.

### Example 1

| | % by weight |
|---|---|
| Petrolatum | 25.0 |
| Calcium sulphate | 6.0 |
| Silica | 3.0 |
| Trllaureth-4 phosphate | 5.0 |
| Fragrance, preservative | q.s. |
| Water | to 100 |

Above composition was prepared by emulsifying petrolatum at elevated temperature with the aid of the surfactant in water and afterwards silica and calcium sulphate were dispersed in the composition and the batch was cooled down to room temperature.

The above composition was applied onto dry hair. It was observed that hair feels soft and smooth and matified. No particles were observed on the hair surface with naked eye.

### Example 2

| | % by weight |
|---|---|
| Petrolatum | 15.0 |
| Ozokerit | 10.0 |
| Calcium sulphate | 5.0 |
| Silica | 4.0 |
| Trilaureth-4 phosphate | 5.0 |
| Ceteareth-20 | 3,0 |
| VPNA copolymer | 3.0 |
| Fragrance, preservative | q.s. |
| Water | to 100 |

The above composition was applied onto dry hair. It was observed that hair feels soft and smooth and matified and well texturized and structured. No particles wore observed on the hair surface with naked eye.

### Example 3

| | % by weight |
|---|---|
| Petrolatum | 15.0 |
| Ozokerit | 10.0 |
| Calcium sulphate | 5.0 |
| Silica | 4.0 |
| Propyleneglycol | 3.0 |
| Tritaureth-4 phosphate | 5.0 |
| PEG-40-Hydrogentaed castor oil | 3.0 |
| VA/Butyl Mateate/tsobomyl | |
| Acrylate Copolymer* | 3.0 |
| Fragrance, preservative | q.s. |
| Water | to 100 |

| | |
|---|---|
| *Advantage Plus from International Specialty products. The concentration in the composition represents active matter. | |

The above composition was applied onto dry hair. It was observed that hair feels soft and smooth and matified and well texturized and structured. No particles were observed on the hair surface with naked eye.

Similar effects were observed with the following examples.

### Example 4

| | % by weight |
|---|---|
| Mineral oil | 15.0 |
| Beeswax | 10.0 |
| Calcium sulphate | 7.0 |
| Silica | 3.0 |
| Trilaureth-4 phosphate | 5.0 |
| PEG-100 Stearate | 2.0 |
| Glyceryl monostearate | 2.0 |
| Panthenol | 0.5 |
| VPNA copolymer | 3.0 |
| Ethylhexylmethoxy cinnamate | 0.5 |
| Fragrance, preservative | q.s. |
| Water | to 100 |

### Example 5

| | % by weight |
|---|---|
| Petrolatum | 15.0 |
| Ozokerit | 10.0 |
| Calcium sulphate | 5.0 |
| Silica | 4.0 |
| Trilaureth-4 phosphate | 5.0 |
| Ceteareth-20 | 3.0 |
| Glycerin | 2.0 |
| VP/VA copolymer | 3.0 |
| Ethanol | 10.0 |
| Fragrance, preservative | q.s. |
| Water | to 100 |

### Example 6

| | % by weight |
|---|---|
| Petrolatum | 15.0 |
| Ozokerit | 5.0 |
| Calcium sulphate | 5.0 |
| Silica | 4.0 |
| Trilaureth-4 phosphate | 5.0 |
| PEG-40 Hydrogenated castor oil | 3.0 |
| Propyleneglycol | 3.0 |
| VPNA copolymer | 3.0 |
| Basic red 51 | 0.1 |
| Ethanol | 10.0 |
| Fragrance, preservative | q.s. |
| Water | to 100 |

### Example 7

| | % by weight |
|---|---|
| Petrolatum | 13.0 |
| Ozokerit | 7.5 |
| Cetearyl alcohol | 2.0 |
| Calcium sulphate | 5.0 |
| Silica | 4.0 |
| Trilaureth- 4 phosphate | 5.0 |
| Ceteareth-20 | 3.0 |
| Propyleneglycol | 3.0 |
| VPNA copolymer | 3.0 |
| Basic yellow 87 | 0.1 |
| Basic red 76 | 0.02 |
| Basic orange 31 | 0.05 |
| Ethanol | 10.0 |
| Fragrance, preservative | q.s. |
| Water | to 100 |

### Example 8

| | % by weight |
|---|---|
| Petrolatum | 15.0 |
| Ozokerit | 10.0 |
| Ceteareth-20 | 3.0 |
| Calcium sulphate | 6.0 |
| Silica | 4.0 |
| Trilaureth-4 phosphate | 3.0 |
| Propyleneglycol | 1.0 |
| VPNA copolymer | 3.0 |
| Bamboo extract | 0.1 |
| Cetrimonium chloride | 0.02 |
| Fragrance, preservative | q.s. |
| Water | to 100 |

### Example 9

| | % by weight |
|---|---|
| Petrolatum | 15.0 |
| Ozokerit | 10.0 |
| Ceteareth-20 | 3.0 |
| Calcium sulphate | 6.0 |
| Silica | 4.0 |
| Trilaureth-4 phosphate | 3.0 |
| Propyleneglycol | 1:0 |
| VP/VA copolymer | 3.0 |
| Bamboo extract | 0.1 |
| Behentrimonium chloride | 0.02 |
| Ethanol | 10.0 |
| Fragrance, preservative | q.s. |
| Water | to 100 |

## Claims

1. An aqueous composition for hair **characterised in that** it comprises at least 20% by weight water, 10 to 60% by weight of at least one wax which is solid at 20°C, at least one emulsifying surfactant at a concentration in the range of 2 to 25% by weight and dispersed particles with a size below 200 µm measured with sieve analysis at a concentration of 1 to 35% by weight, wherein all values are calculated to total composition.

2. Composition according to claim 1 **characterised in that** it comprises as emulsifying surfactant at least one surfactant selected from anionic and non-ionic surfactants.

3. Composition according to claims 1 and 2 **characterised in that** it comprises at least one wax with a melting point above 30°C, more preferably 40°C.

4. Composition according to any of the preceding claims **characterised in that** it comprises first dispersed particles component with a size below 200 µm measured with sieve analysis and a second dispersed particles with the same particle size specification which is selected from the ones inert, non-swelling inorganic powder compounds.

5. Composition according to any of the preceding claims **characterised in that** it comprises the second powder component at a concentration higher than the first dispersed particles.

6. Composition according to any of the preceding claims **characterised in that** it comprises as a first dispersed particle a compound selected from silicium dioxide, starch such as wheat, rice, potato and corn, and titan dioxide and their mixtures.

7. Composition according to any of the preceding claims **characterised in that** it comprises as surfactant a compound according to general structure
(R₃ (OC₂ H₄) ₓ(OC₃ H₆) _{y} O) ₃ PO
wherein R₃ is an alkyl chain which may be saturated or unsaturated branched or straight with 8 to 22 C atoms, preferably 10 to 16 C atoms and more preferably 12 to 16 C atoms, x and y are In the range of 0 to 10 with the condition x+y is more than 0, preferably it is Trilaureth-4 phosphate and its salts.

8. Composition according to any of the preceding claims **characterised in that** it comprises at least one hair styling polymer selected from anionic, cationic. non-ionic and amphoteric ones at a concentration of 0.1 to 25% by weight calculated to total composition.

9. Composition according to any of the preceding claims **characterised in that** it comprises at least one polyol and/or at least one UV filter and/or at least one organic solvent.

10. Composition according to any of the preceding claims **characterised in that** it comprises it comprises at least one propellant.

11. Composition according to any of the preceding claims **characterised in that** it comprises one or more oil at a concentration of 1 to 25% by weight calculated to total of the composition.

12. Composition according to any of the preceding claims **characterised in that** it comprises at least one hair direct dye.

13. Use of composition according to any of the preceding claims for conditioning and/or styling and/or matifying hair.

14. Process for conditioning and/or styling and/or mattifying hair whereín a composition according to claims 1 to 12 is applied onto dry hair.

15. Kit for hair comprising more than one container, preferably 2 to 5, more preferably 2 to 4 and most preferably 2 to 3 containers wherein one of the containers comprises a composition according to claims 1 to 12.
